# EUROPEAN PATENT SPECIFICATION

(11) **EP 3 720 401 B1**
(45) Date of publication and mention of the grant of the patent: **12.01.2022**
(21) Application number: 18822519.7
(22) Date of filing: 03.12.2018
(51) Int. Cl.: A61F 13/04

(54) **A SPLINT-ARMOURED BANDAGE AND A METHOD FOR ITS PRODUCTION**
SCHIENENBEWEHRTE BANDAGE UND VERFAHREN ZU DEREN HERSTELLUNG
BANDAGE-ATTELLE ET SON PROCÉDÉ DE FABRICATION

(30) Priority: 05.12.2017 SE 1700299
(43) Date of publication of application: 14.10.2020
(73) Proprietor: Novortex AB, 740 20 Vänge (SE)
(72) Inventor: LUNDIN, Andreas, 740 20 Vänge (SE)
(86) International application number: PCT/SE2018/051241
(87) International publication number: WO 2019/112508

(56) References cited:
- CN-A- 104 287 894
- CN-Y- 2 369 654
- DE-A1- 2 329 314
- US-A- 2 957 475

## Description

### TECHNICAL FIELD OF THE INVENTION

The present invention relates to a bandage in the form of a strip of fabric with splints attached thereto for use in fixation of an injured or temporarily disabled limb, by wrapping the splint-armoured bandage in layers about the limb. The invention relates also to a method for production of a splint-armoured bandage according to the invention.

### BACKGROUND AND PRIOR ART

A splint-armoured bandage according to the present invention can be seen as a dry alternative to a plaster bandage which needs wetting by water in order to cure and stiffen in applied position about a limb. In comparison, the dry splint-armoured bandage is lightweight, time saving and less sensitive to outer conditions.

The splint-armoured bandage of the present invention can provide a preliminary fixation of an injured or temporary disabled limb or limb joint, until a permanent fixation can be arranged at a hospital or other care unit. The splint-armoured bandage of the present invention can be applied to humans or animals.

Produced in suitable size, the splint-armoured bandage of the present invention can be wrapped into a dressing about an arm, a leg, a finger or a toe, toe and finger joints, ankles, wrists, knees and elbows, for fixation in case of fracture or other temporarily disabling injury. Although explained herein as an item for fixation of limbs or limb joints, the splint-armoured bandage of the present invention may also serve for temporary fixation of other body portions, if appropriate.

In this connection it should be pointed out, that with respect to the occasional use, art and character of the splint-armoured bandage, the present invention belongs to a category of orthopaedic aids which is separated from, e.g., prosthesis, orthoses, liners and compression stockings, which are adapted for wearing on more regular basis.

With respect to its structure, use and functionality, the present invention can be referred to a type of orthopaedic aids as represented by US 2957475 A and DE 2329314 A1, e.g. In both the named cases, rod-shaped substantially straight splints are prefabricated and individually inserted in pockets that are formed in a band or strip of fabric by stitching or sewing, the splints being oriented transversally, i.e. at an angle of about 90°, with respect to the longitudinal direction of the strip of fabric.

A prior splint-armoured bandage and its manufacture is disclosed in CN 104287894 A. In this solution, polymer material splints are individually extruded and applied in situ to a fabric at intermediate angles of more than 0° and less than 90° between adjacent splints, or at angles ranging from more than 0° to less than 90° with respect to the length direction of the bandage.

Whether prefabricated or formed in situ, in the prior art, splint ribs are typically formed and applied individually one-by-one onto a strip of fabric in order to produce a splint-armoured bandage.

### SUMMARY OF THE INVENTION

An object of the present invention is to provide a splint-armoured bandage which in use provides fixation of an injured limb.

Another object of the present invention is to provide a splint-armoured bandage that offers comfort for the wearer.

These objects are met in a splint-armoured bandage wherein radial stiffness and circumferential contraction in a dressing about a limb are balanced so as to provide as good fixation as possible without choking or chafing the treated limb.

Still another object is to design a splint-armoured bandage that permits implementation of an efficient and time saving method for production of the splint-armoured bandage.

To meet with these objects the invention provides, briefly, a splint-armoured bandage comprising at least one flexible splint anchored to a strip of fabric which is elastic in its length direction, the splint having the shape of a wave wherein adjacent splint ribs are interconnected at a wave crest or a wave trough respectively.

More precisely, a bandage for fixation of a limb is disclosed, the bandage comprising at least one flexible, thermoplastic polymer splint which is shaped and in plasticized condition attached to the surface of a longitudinal strip of fabric which is elastic in its length direction, the fabric and splint having a length sufficient for wrapping in layers about a limb. The splint is formed to the shape of waves wherein adjacent splint ribs are interconnected at wave crests and wave troughs respectively, wherein at least one of the wave crests and the wave troughs is located near, i.e. in the region of, a longitudinal edge of the strip of fabric.

In other words, the splint runs wave-shaped on the surface of the strip of fabric, in a plane that is parallel to the plane of the fabric.

An advantage and technical effect of this solution is that when dressed in layers about a wearer's limb, the splint of superimposed bandage layers forms a kind of cross-link network acting as a rigid sleeve. In this sleeve, an outer layer of fabric and splint can be wrapped tightly onto inner layers of fabric and splint without unduly increasing the circumferential contraction of the dressing about the limb, since the splint of the inner layers provide radial and circumferential stiffness to the dressing. This effect results in a firm dressing which avoids choking or chafing of the limb.

Embodiments of the splint include pointed, sinus-shaped, square and trapezoid wave-shapes. Each of these embodiments benefit from the technical effect of a circumferentially continuing element, which adds to rigidity in radial and circumferential directions of a dressing.

In one embodiment, the splint includes a continuous series of splint ribs distributed in the length direction of the bandage, adjacent ribs interconnected at wave crests and wave troughs. This embodiment provides an all-round product adapted for fixation of any kind of fracture or other injury to limbs or limb joints.

In one embodiment, adjacent splints in a series of splints are separated by non-armoured portions of the fabric. This embodiment provides a specialized product adapted for local reduction of the pressure applied from the dressing in a region of the limb.

In one embodiment the splint is anchored to the fabric by being fused or bonded to fibres or threads in the fabric. This embodiment ensures attachment without need for auxiliary fastening steps or arrangements.

In one embodiment, the splint is sandwiched between first and second superimposed layers of fabric. This embodiment secures the splint even further to the fabric. This embodiment also provides an all-textile exterior of the dressing, avoiding the risk of the splint hooking on to external objects.

In embodiments of the invention, the sectional profile of the splint is formed with a width and a height, the height being a normal to the plane and surface of the strip of fabric, and a width to height ratio is the result from subjecting the splint to adjustable compression from a shaping roll when the splint is in plastic state. In some embodiments, the splint is formed with a sectional profile wherein the width to height ratio (w/h) is in the range of about 0.5/1 to about 10/1. In other embodiments, the splint is formed with a sectional profile wherein the width to height ratio (w/h) is in the range of about 1.5/1 to about 4/1. These embodiments provide, in manufacture, control of the elastic character of the splint in radial and circumferential directions of a dressing: a wider splint in the w direction will be stiffer in the plane of the fabric, whereas a splint wider in the h direction will be stiffer transversely to the plane of the fabric.

The objects of the invention are likewise met in a method of producing a splint-armoured bandage, the method comprising:
- heating a thermoplastic polymer material for discharge via a hot extrusion nozzle in molten or semi-molten state,
- feeding a first strip of fabric beneath the hot extrusion nozzle,
- forming a wave-shaped splint on the first strip of fabric by extrusion from the nozzle while moving the nozzle back and forth transversely to the feed of fabric, this way forming a series of splint ribs distributed in the length direction of the strip of fabric and interconnected at their ends by wave crests and wave troughs respectively, and
- positioning at least one of the wave crests and the wave troughs near, i.e. in the region of, a longitudinal edge of the strip of fabric.

The benefits of this method are that splints are shaped and anchored to a strip of fabric in one singular step of production, this way avoiding the preparatory and subsequent work which has hitherto been required by most prior art solutions in splint armoured bandages.

An additional step in the method comprises subjecting the splint to adjustable compression when the splint is passed under a shaping roll in plastic state. The sectional profile of the splint is this way controlled by pressure adjustment to achieve a width to height ratio in the range of about 0.5/1 to about 10/1, preferably to achieve a width to height ratio in the range of about 1.5/1 to about 4/1.

The benefits and technical effects of this manufacturing step are as previously explained.

Other details of the manufacturing method comprise:
- solidification of the splint by feeding the strip of fabric through a cooling region,
- use of a thermosetting, petroleum or cellulose based polymer, with a melting point temperature in the range of 60° to 300° C, and which remains flexible and elastic at temperatures of -20° C or lower,
- use of an elastic fabric web including synthetic or natural fibres, or a mixture or composition thereof, the fabric having a tacky surface,
- applying a second strip of fabric onto the first strip of fabric and splint.
- applying an adhesive to an exposed side of the first or second strip of fabric, wherein the adhesive is chosen to provide adhesion between overlapping layers of fabric.

These and other embodiments of the invention, as well as advantages and technical effects provided by the invention, will also be discussed below in the detailed description and with reference made to the accompanying drawings.

### SHORT DESCRIPTION OF THE DRAWINGS

In the drawings,
- Fig. 1: is a schematic setup of a production line for the manufacture of a splint-armoured bandage according to embodiments of the present invention,
- Fig. 2: is a cut-out detail and sectional view of the production line of Fig. 1 shown on a larger scale,
- Fig. 3: shows a first embodiment of a splint for the splint-armoured bandage,
- Fig. 4: shows the first embodiment of the splint in modified design,
- Fig. 5: shows the splint of the first embodiment expanded in longitudinal direction,
- Fig. 6: is a schematic representation of overlapping layers of splint-armoured bandage,
- Fig. 7: is an elevation view showing the splint-armoured bandage of the present invention dressed about a human foot and ankle,
- Fig. 8: shows an alternative splint design,
- Fig. 9: shows another alternative splint design,
- Fig. 10: shows yet another design of the splint,
- Fig. 11: shows an alternative application of splints, and
- Fig. 12: shows yet an alternative design of the splint-armoured bandage.

### DETAILED DESCRIPTION OF DISCLOSED EMBODIMENTS

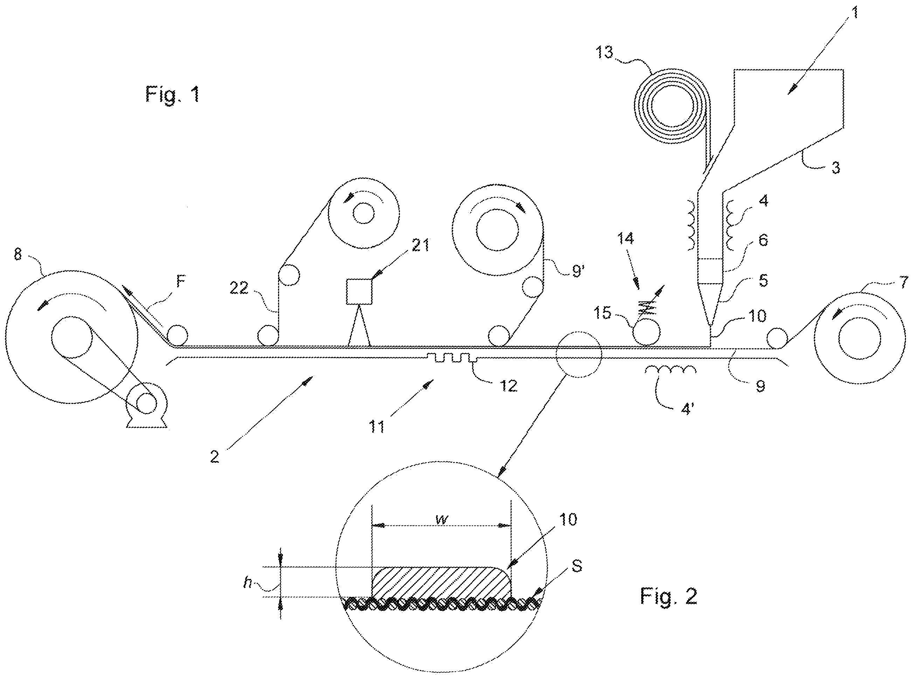

With reference to Fig. 1, a production line for the manufacture of a splint-armoured bandage by application of a splint to a longitudinal strip of fabric comprises a hot extrusion station 1 and a fabric feeding path 2.

The hot extrusion station 1 includes a container 3 for a thermoplastic polymer material, equipped with a heater 4 and a hot extrusion nozzle 5. One or more additional heaters 4' may be installed along the fabric path 2 in order to maintain or return the splint into plastic state.

The hot extrusion nozzle 5 is suspended movable above the fabric feeding path 2 and controlled in back and forth movements, transversely to the fabric feeding path 2, by means of a nozzle control 6. Nozzle control also involves controlling the discharge rate of polymer product via the nozzle. The specification of components in the hot extrusion station 1 may correspond generally to those conventionally applied in extrusion of polymer and co-polymer materials.

The fabric feeding path 2 defines a feed direction F from a fabric supply reel 7 to a reel 8 for a splint-armoured fabric. The reel 8 is driven for feeding, continuously or intermittently as the case may be, a strip of fabric 9 from the supply reel 7 to pass underneath the nozzle 5 from which a string of molten or semi-molten polymer product 10 is discharged so as to form a splint 10 on the fabric. The path length from the nozzle 5 to the reel 8 is long enough to permit cooling and solidification of the splint, until the fabric with splint is rolled onto the reel 8. For the purpose of accelerating solidification, the fabric feeding path 2 may include a cooling region 11 through which the fabric and splint passes before it reaches the reel 8. The cooling region may include a cooling fan, or a cooling plate 12, e.g., over which the fabric and splint is passed in the feed direction F.

A fabric suitable for production of a splint-armoured bandage according to the present invention can be comprised of synthetic fibres or natural fibres, or a mix or composition of synthetic and natural fibres. A composite fibre may include a cotton fibre or thread coated with an elastomeric compound, e.g. The fibres may be formed into threads that are woven, knitted or interlaced to produce the fabric. At least the warp or yarn of the fabric is elastic. The elasticity may result from intrinsic properties in a synthetic, composite or rubber thread, and may alternatively be the result of appropriate knitting or weaving technique.

A suitable fabric is a fabric comprising fibres or threads that provide a tacky effect. The character of tackiness which is aimed for results in self-adhesion between superimposed layers of fabric in a dressing about a limb. Fabric which meets the desired tackiness and elasticity can be found commercially, such as the brands of fabric which are conventionally used in adhesive bandages and wound dressings.

A polymer suitable for production of a splint-armoured bandage according to the present invention is a thermoplastic or thermosetting polymer with a melting point temperature that is well above the upper temperatures prevailing in normal use of the bandage. At the other end of the scale, the temperature of the polymer splint in molten or semi-molten state must not deform or damage the fibres or threads in the fabric. Particular attention should be paid to specifications, such as melting point temperature, for synthetic fibres included in the fabric. It is also essential that the splint material stays flexible, i.e. does not become brittle, at the lower temperatures prevailing in normal use of the bandage. A thermoplastic or thermosetting polymer which has a melting point temperature within a range of 60° to 300° C, and which remains flexible and elastic at temperatures of -20° C or lower, is considered to meet the purpose for a wide range of embodiments.

Without excluding other alternatives, a splint material according to specifications may be found among, e.g., polyethylene, polypropylene, polyvinyl chloride, polyamide, or among thermoplastic cellulose derivatives such as cellulose acetate e.g., with or without plasticizer added to the polymer composition.

The manufacturing process can include a pelletized or granulated polymer material which is heated to melting temperature for discharge via the nozzle 5.

Alternatively, the manufacturing process can include discharge of a solid polymer thread 13 (see Fig. 1) which is heated to a temperature below melting temperature (semi-molten) at which the thread can be plastically deformed and its applied shape on the fabric be maintained after cooling. In one embodiment, the splint is shaped from a solid polymer thread, such as a polyamide or nylon thread, with a homogenous section and a diameter of about 0.5 - 10 mm. However, whether starting from a pelletized material or a thread, in either alternative the splint can be shaped and applied to the strip of fabric in a plasticized condition, and in solidified state attaching to the threads and/or fibres in the fabric, without requiring special preparation or modification of the fabric.

It shall be noted that the shape and dimensions of the splint can be varied through proper control of fabric feed rate and polymer discharge rate at the hot extrusion nozzle 5. With reference to Fig. 2, which is a cut-out detail and sectional view of the production line of Fig. 1 shown on a larger scale, a transverse section of a splint 10 is formed with a width w and a height h, the height h being a normal to the plane and surface S of the strip of fabric.

The width to height ratio in the sectional profile of the splint 10 is the result from subjecting the splint to a controlled compression applied in a compression region 14, arranged in the production line (see Fig. 1) at a position where the splint 10 is still in plastic state. The compression region comprises a shaping roll 15 under which the fabric and splint is passed in the feed direction. By adjustment of the pressure applied from the shaping roll 15 there is accomplished control of stiffness of the splint in the directions w and h. That is, a wider splint in the w direction will be stiffer in the longitudinal plane of the fabric (i.e., in the circumferential direction of a dressing about a limb), whereas a splint wider in the h direction will be stiffer transversely to the plane of the fabric (i.e. in radial direction of a dressing about a limb).

According to this aspect of the present invention, and more precisely by properly adjusting the height of a gap between the shaping roll 15 and the fabric 9, the section of the splint 10 can be formed with various w/h ratios.

It is possible and lies within the scope of the invention to arrange the mouth of the nozzle 5 such that a polymer string is deposited onto the fabric, wherein the width w of the splint section is less than the height h of the splint section. This can be achieved, for example, using a rectangular nozzle mouth which is tilted at an angle of about 45° from the plane of the fabric. The w/h ratio may this way be extended to a lower range limit of about 0.5/1, at least. An upper range limit, up to which the splint will still provide adequate radial support in a dressing, is for this practical reason defined at a w/h ratio of about 10/1.

The applicant has found that a width to height ratio within the range of about 1.5/1 to about 4/1 provides a most beneficiary balance between elasticity and radial and circumferential rigidity in a dressing, and is therefore preferred in a bandage which is armoured by means of a wave-shaped polymer splint.

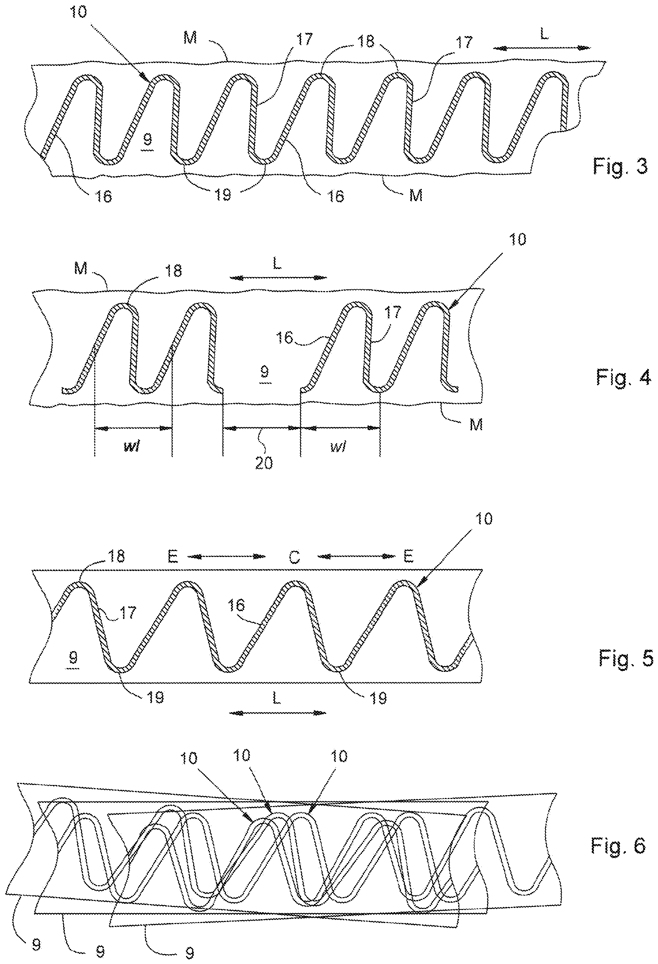

One embodiment of a splint-armoured bandage produced in a production line substantially as recited above is shown in Fig. 3. In this embodiment, the splint 10 includes a continuous series of splint ribs 16 and 17 distributed in the length direction L of the strip of fabric 9, and interconnected at their ends by wave crests 18 and wave troughs 19 respectively.

More precisely, a discrete length of the splint-armoured bandage may include one singular wave-shaped splint 10 extended the full length of the bandage, as illustrated in Fig. 3.

In one embodiment, a splint-armoured bandage may include a series of splints 10 of shorter lengths, wherein adjacent splints are separated in the longitudinal direction L by a non-armoured length 20 of the bandage, as illustrated in Fig. 4.

In particular, the shortest wave-forming element wl available in a splint-armoured bandage according to the present invention is one wavelength long, including a pair of adjacent splint ribs 16 and 17, in one of their ends interconnected by a wave crest 18 or a wave trough 19 respectively.

With reference to Fig. 5 a splint 10, whether produced in a petroleum based or cellulose based thermoplastic polymer, provides in solidified state a flexible and elastic armour that follows in elongation E and contraction C of the fabric which is elastic in its length direction L. The elastic connection between adjacent splint ribs, i.e. at wave crests and wave troughs, results in a splint acting like a tension spring element which operates in the length direction of the fabric, permitting elongation and adding to contraction of the fabric from an expanded state.

In a splint-armoured bandage according to the present invention, the strip of fabric 9 and the splint 10 have a length L sufficient for wrapping in layers about a limb. Without limiting the invention to a specified length, but rather to indicate the art and character of the subject of the present invention, a length L in the range of 0.5 to 10 meters is considered to cover most practical cases. If appropriate, a length of fabric in one or both ends of the fabric may be left without the splint in order to facilitate the foundation and/or finishing of a dressing about a limb. Excessive lengths of the splint-armoured bandage can be cut off from a dressing by using knife or scissors.

The strip of fabric 9 may be formed with transverse widths in the order of about 10 mm up to the order of about 400 mm, covering various needs for fixation of body portions the size of an upper body down to finger size.

In one embodiment of the present invention, the splint ribs are formed with a w/h ratio which differs from the w/h ratios of the splint waves or the splint crests. For example, by applying pressure only in the longitudinal edge regions of the splint-armoured fabric, the wave crests 18 and/or the wave troughs 19 can be flattened to a higher w/h ratio, such as 1.5/1 or higher, whereas the splint ribs 16 and 17 are left at a comparatively lower w/h ratio, such as 0.5/1 or higher. This way, elasticity in the length direction of the splint-armoured fabric can be altered without affecting the radial stiffness transversally to the fabric.

The frequency of waves in a dressing, and thus the density of armour, can also be adjusted and adopted to need and anatomy in the treated limb by more or less stretching of fabric and splint.

With reference to Fig. 6, the radial and circumferential rigidity in a dressing can also be enhanced locally by arranging crossing splints in overlapping layers of fabric, such as at the location of a suspected fracture, e.g.

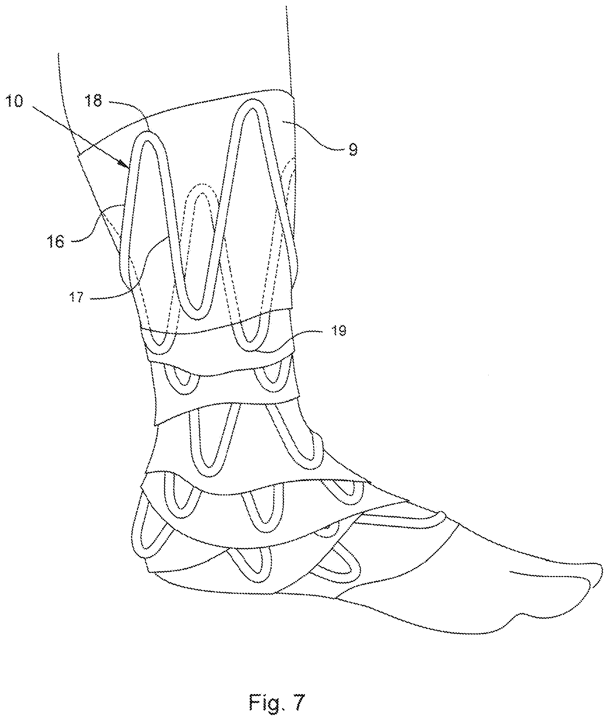

Fig. 7 shows the splint-armoured bandage of Figs. 3-6 dressed about a human ankle. From the drawing of Fig. 7 it can be appreciated that in applied state the splint-armoured bandage forms an overlapping and crosslinked network of splints that provide all-around support, in appearance similar to scales in the panzer of a fish or reptile. Yet, the armour is still flexible to avoid discomfort caused by pressure or chafing, and permits adaptation to protrusions on the limb, such as heel and fibula e.g., which can be positioned between splint ribs 16, 17.

In the manufacturing process, feeding the strip of fabric as well as nozzle movement and polymer discharge rate may be controlled for continuous or intermittent operation. Different splint shapes and patterns can this way be formed onto the fabric.

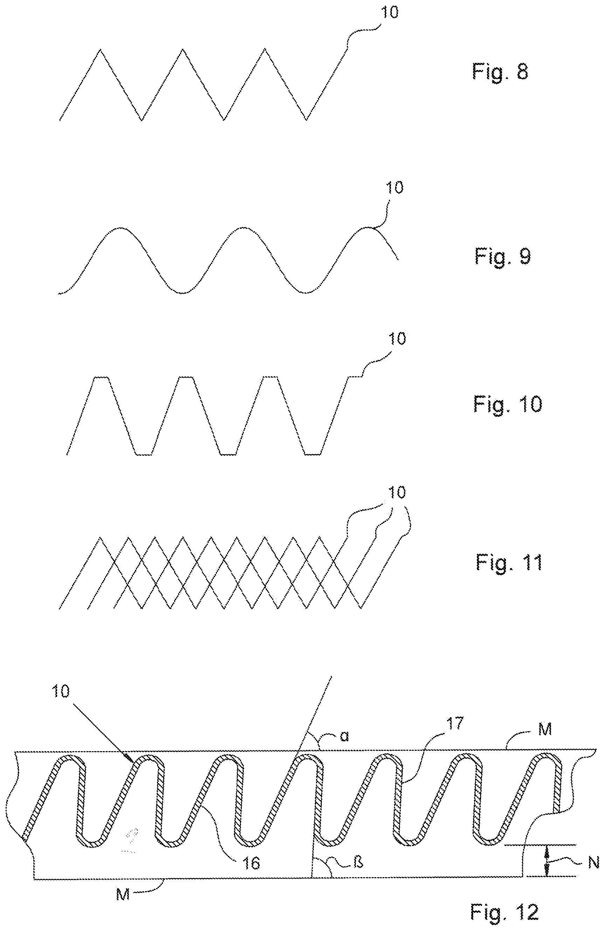

A sample of splint shapes and embodiments made available by the present invention are illustrated in Figs. 8-11. In Fig. 8 the splint has a pointed wave shape, whereas in Fig. 9 the wave shape is sinusoidal. Fig. 10 illustrates a splint having the shape of a square or trapezoidal wave. With respect to its shape, a common feature of disclosed embodiments is that the splint is shaped as a wave extended in a plane parallel to the plane of the fabric, the splint changing its run direction, uninterrupted, in a wave crest or a wave trough respectively.

The manufacturing process can be modified. For example, the feed direction of the fabric may be reversed in a process of applying one or more additional splints. In a set of splints, the splints may be arranged to run in parallel side-by-side while displaced laterally in the width direction of the strip of fabric, or the splints can be arranged intersecting each other. Alternatively, two or more hot extrusion stations may be arranged in succession in the production line to produce parallel or intersecting splint patterns, such as the one illustrated in Fig. 11, e.g.

The distribution of the splint 10 extends generally in longitudinal direction of the strip of fabric, the wave crests and troughs located near the longitudinal margins M of the strip of fabric (see Fig. 12).

However, the transverse width or amplitude of the splint need not cover the full width of the strip of fabric: in fact, a portion N of the width of the fabric may be left without armour in the length of the fabric, substantially as illustrated in Fig. 12. Although not illustrated in the drawings, it will be understood that the splint or splints may alternatively be centred on the strip of fabric, leaving unarmoured corridors along both edges of the fabric. Preferably though, at least one of the wave crests and the wave troughs is located near, i.e. in the region of, a longitudinal edge M of the strip of fabric as illustrated in Fig. 12.

It serves to be noted, still with reference to Fig. 12, that the inclinations α, β of the splint ribs 16 and 17 with respect to the longitudinal direction of the strip of fabric 9 need not be the same for the full length of the strip of fabric. By adjusting the feeding speed of the fabric and/or speed of movements of the hot extrusion nozzle, the ribs 16, 17 following each other in succession may be given different inclinations with respect to the fabric longitudinal direction. By varying the inclination of splint ribs there is achieved a possibility of concentrating the stabilizing forces in a desired direction in a bandage dressing. This feature can be of advantage, e.g., in the fixation of a joint at an angle between upper and lower limb portions.

In a splint-armoured bandage produced and composed substantially as described above, the splint may be anchored to the fabric through bonding at the interface between the splint and the fabric. In this context, "bonding" is used as a definition of a connection between splint and fabric that arises upon solidification of splint material which in molten or semi-molten phase has adhered to threads or fibres in the fabric. Bonding can also comprise penetration of molten splint material into and/or between threads or fibres in the fabric. If appropriate, bonding may also include fusion between the splint material and any synthetic component in the fabric. Bonding between splint and fabric can be enhanced when splint material is forced into the fabric during the step of compressing the splint while in plastic state as mentioned above.

However, a splint may be anchored and affixed to the fabric in other ways beside bonding.

With reference again to Fig. 1, finishing of the splint-armoured bandage optionally involves application of an additional second strip of fabric 9' which is arranged superimposed on the first strip of fabric and splint. The second strip of fabric can be introduced in the fabric feeding path 2 at a position downstream of the shaping roll 15 as illustrated, or at a position (not illustrated) upstream of the shaping roll 15. The resulting bandage forms a laminate wherein the splint is anchored in the bandage by being sandwiched between layers of fabric.

The layers of fabric may be secured to each other simply through the adhesion between tacky fabric surfaces being laid or pressed together. Alternatively, or in addition thereto, stitches of thread may be applied through the layers of fabric. Stitching may also serve for fixation of the splint in a laminate of superimposed layers of fabric. Other alternatives include application of heat and/or pressure to achieve connection between the layers of fabric. If appropriate, a lamination station (not shown in the drawings) performing sewing, heating or compression may be included in the production line of Fig. 1.

Beside the embodiments presented above, the invention can be modified and realized in other ways without abandoning the scope of the invention.

One modification of the invention comprises an additional step in the production process, wherein an external layer, or string, of adhesive is applied to the outer and exposed side of the second strip of fabric 9'. The purpose of the added adhesive layer is to improve adhesion between overlapping layers of fabric, this way even further increasing the integrity of a dressing applied to a limb. With reference again to Fig. 1, the laminate of the first and second fabric with the splint positioned there-between can be fed through an adhesive transfer station 21, comprising an adhesive transfer roll or an adhesive spray curtain, e.g. At a position downstream of the location of the station 21, the laminate can be arranged to receive a protective film or paper 22 before the finished product is wound onto the reel 8.

It will be realized that a splint of wave-shape may alternatively be prefabricated in a heat extrusion process and stored on a reel. A splint of wave-shape may also alternatively be prefabricated from a sheet of material by punching, or even produced from a solid thread that is bent into wave-shape. The prefabricated splint is then, in solid state, rolled off onto a strip of fabric moving down the fabric feeding path. By heating the splint to thermoforming temperature the splint is set in plastic state, its sectional profile shaped and bonded to the fabric by application of pressure from a shaping roll as previously explained.

Accompanying claims define the invention as described and explained, including disclosed embodiments and others which would be readily derivable from the disclosure although not explicitly illustrated and specified herein.

## Claims

1. A bandage for fixation of a limb, the bandage comprising at least one flexible, thermoplastic polymer splint (10) which is shaped and in plasticized condition attached to the surface (S) of a longitudinal strip of fabric (9) which is elastic in its length direction (L), the fabric (9) and splint (10) having a length (L) sufficient for wrapping in layers about a limb, **characterized in that** the splint (10) is formed to the shape of waves wherein adjacent splint ribs (16, 17) are interconnected at wave crests (18) and wave troughs (19) respectively, wherein at least one of the wave crests (18) and the wave troughs (19) is located near, i.e. in the region of, a longitudinal edge (M) of the strip of fabric (9).

2. The bandage of claim 1, wherein the wave-shape of the splint (10) is one of pointed, sinus-shaped, square and trapezoid.

3. The bandage of claim 1 or 2, wherein a splint (10) includes a continuous series of splint ribs (16, 17) distributed in the length direction (L) of the bandage, the splint ribs interconnected by wave crests and wave troughs (18, 19).

4. The bandage of claim 1 or 2, wherein in a series of splints, adjacent splints (10) are separated by non-armoured portions (20) of the fabric.

5. The bandage of any previous claim, wherein the splint (10) is anchored to the fabric (9) by being fused or bonded to fibres or threads in the fabric.

6. The bandage of any previous claim, wherein the splint (10) is sandwiched between first (9) and second (9') superimposed layers of fabric.

7. The bandage of any previous claim, wherein a sectional profile of the splint (10) is formed with a width (w) and a height (h), the height (h) being a normal to the plane and surface (S) of the strip of fabric (9), and a width to height ratio (w/h) in the sectional profile of the splint is the controlled result from subjecting the splint to adjustable compression from a shaping roll (15) when the splint is in plastic state.

8. The bandage of claim 7, wherein the sectional profile of the splint (10) has a width to height ratio (w/h) in the range of about 0.5/1 to about 10/1.

9. The bandage of claim 8, wherein the sectional profile of the splint (10) has a width to height ratio (w/h) in the range of about 1.5/1 to about 4/1.

10. A method of producing an armoured bandage for fixation of a limb, the method comprising
a. heating a thermoplastic polymer material for discharge via a hot extrusion nozzle in molten or semi-molten state,
b. feeding a first strip of fabric beneath the hot extrusion nozzle,
c. forming a wave-shaped splint on the first strip of fabric by extrusion from the nozzle while moving the nozzle back and forth transversely to the feed of fabric, this way forming a series of splint ribs (16; 17) distributed in the length direction (L) of the strip of fabric (9) and interconnected at their ends by wave crests (18) and wave troughs (19) respectively, and
d. positioning at least one of the wave crests (18) and the wave troughs (19) near, i.e. in the region of, a longitudinal edge (M) of the strip of fabric.

11. The method of claim 10, comprising the step of controlling a width to height ratio (w/h) in the sectional profile of the splint by subjecting the splint to adjusted compression when the splint is passed in plastic state under a shaping roll (15).

12. The method of claim 11, comprising the step of forming, by pressure adjustment, the sectional profile of the splint (10) with a width to height ratio (w/h) in the range of about 0.5/1 to about 10/1.

13. The method of claim 12, comprising the step of forming, by pressure adjustment, the sectional profile of the splint (10) with a width to height ratio (w/h) in the range of about 1.5/1 to about 4/1.

14. The method of any of claims 10-13, wherein solidification of the splint is enhanced by feeding the strip of fabric through a cooling region.

15. The method of any of claims 10-14, wherein the polymer material is selected from thermoplastic or thermosetting, petroleum or cellulose based polymers, with a melting point temperature in the range of 60° to 300° C, and which remains flexible and elastic at temperatures of -20° C or lower.

16. The method of any of claims 10-15, wherein the fabric selected is an elastic web including synthetic or natural fibres, or a mixture or composition thereof, the fabric having a tacky surface.

17. The method of any of claims 10-16, comprising the step of applying a second strip of fabric onto the first strip of fabric and splint.

18. The method of claim 17, comprising the step of applying an adhesive to an exposed side of the first or second strip of fabric, wherein the adhesive is chosen to provide adhesion between overlapping layers of fabric.

## Patentansprüche

1. Bandage zur Fixierung einer Gliedmaße, wobei die Bandage mindestens eine flexible, thermoplastische Polymerschiene (10) umfasst, die geformt ist und in plastifizierter Kondition an der Oberfläche (S) eines Längsstreifens aus Stoff (9), der in seiner Längsrichtung (L) elastisch ist, angebracht ist, wobei der Stoff (9) und die Schiene (10) eine Länge (L) aufweisen, die für das Wickeln in Schichten um eine Gliedmaße ausreicht, **dadurch gekennzeichnet, dass** die Schiene (10) in Wellenform gebildet ist, wobei benachbarte Schienenlamellen (16, 17) durch Wellenberge (18) bzw. Wellentäler (19) miteinander verbunden sind, wobei sich mindestens einer der Wellenberge (18) und eines der Wellentäler (19) in der Nähe, d. h. im Bereich eines Längsrandes (M) des Stoffstreifens (9), befindet.

2. Bandage nach Anspruch 1, wobei die Wellenform der Schiene (10) eine von spitz, sinusförmig, quadratisch und trapezoid ist.

3. Bandage nach Anspruch 1 oder 2, wobei eine Schiene (10) eine fortlaufende Reihe von Schienenlamellen (16, 17) einschließt, die in der Längsrichtung (L) der Bandage verteilt sind, wobei die Schienenlamellen durch Wellenberge bzw. Wellentäler (18, 19) miteinander verbunden sind.

4. Bandage nach Anspruch 1 oder 2, wobei benachbarte Schienen (10) in einer Reihe von Schienen durch nicht armierte Abschnitte (20) des Stoffs getrennt sind.

5. Bandage nach einem der vorstehenden Ansprüche, wobei die Schiene (10) an dem Stoff (9) verankert ist, indem sie mit Fasern oder Fäden in dem Stoff fusioniert oder gebondet ist.

6. Bandage nach einem der vorstehenden Ansprüche, wobei die Schiene (10) zwischen der ersten (9) und der zweiten (9') übereinander gelegten Stoffschicht angeordnet ist.

7. Bandage nach einem der vorstehenden Ansprüche, wobei ein Querschnittsprofil der Schiene (10) mit einer Breite (w) und einer Höhe (h) gebildet ist, wobei die Höhe (h) eine Normale zur Ebene und Oberfläche (S) des Stoffstreifens (9) ist und ein Verhältnis der Breite zur Höhe (w/h) in dem Querschnittsprofil der Schiene das gesteuerte Ergebnis des Unterziehens der Schiene einer einstellbaren Kompression durch eine Formgebungswalze (15) ist, wenn die Schiene in einem plastischen Zustand ist.

8. Bandage nach Anspruch 7, wobei das Querschnittsprofil der Schiene (10) ein Verhältnis der Breite zur Höhe (w/h) im Bereich von etwa 0,5/1 bis etwa 10/1 aufweist.

9. Bandage nach Anspruch 8, wobei das Querschnittsprofil der Schiene (10) ein Verhältnis der Breite zur Höhe (w/h) im Bereich von etwa 1,5/1 bis etwa 4/1 aufweist.

10. Verfahren zum Herstellen einer armierten Bandage zur Fixierung einer Gliedmaße, wobei das Verfahren Folgendes umfasst
a. Erwärmen eines thermoplastischen Polymermaterials zur Abgabe über eine Heißextrusionsdüse in geschmolzenem oder halbgeschmolzenem Zustand,
b. Zuführen eines ersten Stoffstreifens unter die Heißextrusionsdüse,
c. Bilden einer wellenförmigen Schiene auf dem ersten Stoffstreifen durch Extrusion aus der Düse, während die Düse quer zur Zuführung des Stoffs vor und zurück bewegt wird, wodurch eine Reihe von Schienenlamellen (16, 17) gebildet wird, die in der Längsrichtung (L) des Stoffstreifens (9) verteilt und an ihren Enden durch Wellenberge (18) bzw. Wellentäler (19) miteinander verbunden sind, und
d. Positionieren mindestens eines/einer der Wellenberge (18) und der Wellentäler (19) in der Nähe, d. h. im Bereich eines Längsrandes (M) des Stoffstreifens.

11. Verfahren nach Anspruch 10, umfassend den Schritt des Steuerns eines Verhältnisses der Breite zur Höhe (w/h) in dem Querschnittsprofil der Schiene durch Unterziehen der Schiene einer eingestellten Kompression, wenn die Schiene im plastischen Zustand unter einer Formgebungswalze (15) hindurchgeführt wird.

12. Verfahren nach Anspruch 11, umfassend den Schritt des Bildens, durch Druckeinstellung, des Querschnittsprofils der Schiene (10) mit einem Verhältnis der Breite zur Höhe (w/h) im Bereich von etwa 0,5/1 bis etwa 10/1.

13. Verfahren nach Anspruch 12, umfassend den Schritt des Bildens, durch Druckeinstellung, des Querschnittsprofils der Schiene (10) mit einem Verhältnis der Breite zur Höhe (w/h) im Bereich von etwa 1,5/1 bis etwa 4/1.

14. Verfahren nach einem der Ansprüche 10-13, wobei das Erstarren der Schiene durch Leiten des Stoffstreifens durch einen Kühlbereich verstärkt wird.

15. Verfahren nach einem der Ansprüche 10-14, wobei das Polymermaterial aus thermoplastischen oder duroplastischen erdöl- oder cellulosebasierten Polymeren mit einer Schmelzpunkttemperatur im Bereich von 60 °C bis 300 °C ausgewählt ist und bei Temperaturen von -20 °C oder darunter flexibel und elastisch bleibt.

16. Verfahren nach einem der Ansprüche 10-15, wobei der Stoff aus einem elastischen Gewebe ausgewählt ist, das synthetische oder natürliche Fasern oder eine Mischung oder Zusammensetzung davon einschließt, wobei der Stoff eine klebrige Oberfläche aufweist.

17. Verfahren nach einem der Ansprüche 10-16, umfassend den Schritt des Aufbringens eines zweiten Stoffstreifens auf den ersten Stoffstreifen und die Schiene.

18. Verfahren nach Anspruch 17, umfassend den Schritt des Aufbringens eines Haftmittels auf eine exponierte Seite des ersten oder zweiten Stoffstreifens, wobei das Haftmittel gewählt ist, um Haftung zwischen sich überlappenden Stoffschichten bereitzustellen.

## Revendications

1. Bandage pour l'immobilisation d'un membre, le bandage comprenant au moins une attelle polymère thermoplastique souple (10) qui est façonnée et fixée à l'état recouvert de plastique à la surface (S) d'une bande de tissu longitudinale (9) qui est élastique dans le sens de sa longueur (L), le tissu (9) et l'attelle (10) présentant une longueur (L) suffisante pour s'enrouler en plusieurs couches autour d'un membre, **caractérisé en ce que** l'attelle (10) prend la forme de vagues dans lequel des nervures d'attelle adjacentes (16, 17) sont reliées entre elles au niveau de crêtes de vague (18) et de creux de vague (19) respectivement, dans lequel au moins l'un parmi les crêtes de vague (18) et les creux de vague (19) est situé proche, c'est-à-dire dans la région, d'un bord longitudinal (M) de la bande de tissu (9).

2. Bandage selon la revendication 1, dans lequel la forme de vague de l'attelle (10) est l'une parmi pointue, de forme sinusoïdale, carrée et trapézoïdale.

3. Bandage selon la revendication 1 ou 2, dans lequel une attelle (10) inclut une série continue de nervures d'attelle (16, 17) réparties dans le sens de la longueur (L) du bandage, les nervures d'attelle étant reliées entre elles par des crêtes de vague et des creux de vague (18, 19).

4. Bandage selon la revendication 1 ou 2, dans lequel dans une série d'attelles, des attelles adjacentes (10) sont séparées par des parties non renforcées (20) du tissu.

5. Bandage selon une quelconque revendication précédente, dans lequel l'attelle (10) est ancrée au tissu (9) en étant fusionnée ou liée à des fibres ou à des fils dans le tissu.

6. Bandage selon une quelconque revendication précédente, dans lequel l'attelle (10) est prise en sandwich entre une première couche superposée (9) et une seconde couche superposée (9') de tissu.

7. Bandage selon une quelconque revendication précédente, dans lequel un profil transversal de l'attelle (10) est pourvu d'une largeur (w) et d'une hauteur (h), la hauteur (h) étant une perpendiculaire au plan et une surface (S) de la bande de tissu (9), et un rapport entre largeur et hauteur (w/h) dans le profil transversal de l'attelle est le résultat régulé de la soumission de l'attelle à une compression ajustable d'un rouleau de mise en forme (15) lorsque l'attelle est dans l'état plastique.

8. Bandage selon la revendication 7, dans lequel le profil transversal de l'attelle (10) présente un rapport entre largeur et hauteur (w/h) dans la plage d'environ 0,5/1 à environ 10/1.

9. Bandage selon la revendication 8, dans lequel le profil transversal de l'attelle (10) présente un rapport entre largeur et hauteur (w/h) dans la plage d'environ 1,5/1 à environ 4/1.

10. Procédé de production d'un bandage renforcé pour l'immobilisation d'un membre, le procédé comprenant
a. le chauffage d'un matériau polymère thermoplastique en vue de sa distribution par l'intermédiaire d'une buse d'extrusion à chaud dans un état fondu ou semi fondu,
b. l'acheminement d'une première bande de tissu sous la buse d'extrusion à chaud,
c. le façonnage d'une attelle en forme de vagues sur la première bande de tissu par extrusion depuis la buse tout en déplaçant la buse d'avant en arrière de manière transversale à l'acheminement de tissu, façonnant ainsi une série de nervures d'attelle (16 ; 17) réparties dans le sens de la longueur (L) de la bande de tissu (9) et reliées entre elles à leurs extrémités par des crêtes de vague (18) et des creux de vague (19) respectivement, et
d. le positionnement d'au moins l'un parmi les crêtes de vague (18) et les creux de vague (19) proche, c'est-à-dire dans la région, d'un bord longitudinal (M) de la bande de tissu.

11. Procédé selon la revendication 10, comprenant l'étape de régulation d'un rapport entre largeur et hauteur (w/h) dans le profil transversal de l'attelle par soumission de l'attelle à une compression ajustée lorsque l'attelle est passée dans l'état plastique sous un rouleau de mise en forme (15).

12. Procédé selon la revendication 11, comprenant l'étape de façonnage, par ajustement de pression, du profil transversal de l'attelle (10) avec un rapport entre largeur et hauteur (w/h) dans la plage d'environ 0,5/1 à environ 10/1.

13. Procédé selon la revendication 12, comprenant l'étape de façonnage, par ajustement de pression, du profil transversal de l'attelle (10) avec un rapport entre largeur et hauteur (w/h) dans la plage d'environ 1,5/1 à environ 4/1.

14. Procédé selon l'une quelconque des revendications 10-13, dans lequel une solidification de l'attelle est accrue par acheminement de la bande de tissu à travers une région de refroidissement.

15. Procédé selon l'une quelconque des revendications 10-14, dans lequel le matériau polymère est sélectionné parmi des polymères thermoplastiques ou thermodurcissables, à base de pétrole ou de cellulose, avec une température de point de fusion dans la plage de 60 ° à 300 °C, et qui reste souple et élastique à des températures inférieures ou égales à -20 °C.

16. Procédé selon l'une quelconque des revendications 10-15, dans lequel le tissu sélectionné est un voile élastique incluant des fibres synthétiques ou naturelles, ou un mélange ou une composition de celles-ci, le tissu présentant une surface collante.

17. Procédé selon l'une quelconque des revendications 10-16, comprenant l'étape d'application d'une seconde bande de tissu sur la première bande de tissu et l'attelle.

18. Procédé selon la revendication 17, comprenant l'étape d'application d'un adhésif sur un côté visible de la première ou seconde bande de tissu, dans lequel l'adhésif est choisi pour fournir une adhérence entre des couches superposées de tissu.
